# EUROPEAN PATENT APPLICATION

(11) **EP 3 848 939 A1**
(43) Date of publication of application: **14.07.2021**
(21) Application number: 20150537.7
(22) Date of filing: 07.01.2020
(51) Int. Cl.: G16H 30/40, G06N 20/00, G16H 50/20, G16H 50/70

(54) **PROCESSING A CLASSIFIER**

(71) Applicant: Robert Bosch GmbH, 70442 Stuttgart (DE)
(72) Inventor: Schiegg, Martin, 70825 Korntal-Muenchingen (DE); Gerwinn, Sebastian, 71229 Leonberg (DE); Zimmer, Christoph, 70825 Korntal (DE); Zafar, Muhammad Bilal, 71272 Renningen (DE); Rudolph, Maja Rita, 72072 Tuebingen (DE)

(57) **Abstract**

The invention relates to a system (100) for processing a classifier. The classifier is a Naïve Bayes-type classifier classifying an input instance into multiple classes based on multiple continuous probability distributions of respective features of the input instance and based on prior probabilities of the multiple classes. Upon receiving a removal request message identifying one or more undesired training instances, the classifier is made independent from one or more undesired training instances. To this end, for a continuous probability distribution of a feature, adapted parameters of said probability distribution are computed based on current parameters of said probability distribution and the one or more undesired training instances. Further, an adapted prior probability of a class is computed based on a current prior probability of the class and the one or more undesired training instances.

## Description

### FIELD OF THE INVENTION

The invention relates to a system for processing a classifier, and to a corresponding computer-implemented method. The invention further relates to a computer-readable medium comprising instructions to perform the method.

### BACKGROUND OF THE INVENTION

Wearable devices such as smart watches, fitness trackers, and body-mounted sensors allow to measure and track various quantities of a user, for example, physiological quantities such as heart rate or blood pressure, or other kinds of physical quantities such as location, speed, rotational velocity, etcetera. Such measurements, which are typically represented by continuous features, are then typically centrally collected and various services can be provided that make use of the measurements, for example, activity logging, sleep advise, etcetera. Many of these services apply classification models to the information collected from users, for example, to recognize patterns or to detect anomalies. One class of classification models that can be applied to such continuous features is the class of Naive Bayes classifiers. Such a classifier may classify an input instance into multiple classes, e.g., assign one of the multiple classes to the input instance, based on continuous probability distributions of the respective features of the input instance and based on prior probabilities of the multiple classes. These classifiers are popular due to their efficiency and their ability to assign probabilities to respective classes for an input instance. Apart from applying models to the information collected from users, services usually also use this information to further refine the machine learning models and thus improve their services. Also in many other settings, classification models are trained on personal information, for example, in medical image processing or facial recognition.

If a classifier is trained on a training dataset including personal information about a certain person, then this means that the machine learning model is dependent on that personal information in the sense that, if this personal information would not have been included in the dataset, the training would have led to a different model. In particular, the set of parameters of the classifier may be different. As a consequence, also for at least one input instance to which the classifier may be applied, the model trained using the personal information may provide a different model output from the model trained without the personal information. In some cases, due to these differences, it turns out to be possible to derive information about individuals in a dataset just from the model, a phenomenon known as "model inversion". More generally, since a classifier is effectively a function of the training dataset including their personal information, it would be desirable if a classifier could, if persons included in the training dataset so desired, be made substantially independent from training instances involving them. In fact, in many settings privacy regulations such as the General Data Protection Regulation (GDPR) of the European Union or the Health Insurance Portability and Accountability Act (HIPPA) of the United States may require up to various degrees to let a data subject control to what extent their personal information may be used, for example, to train machine learning models.

A known way of limiting the dependence of model outputs on any one particular training record is by making use of differentially private perturbation techniques. Differential privacy is a mathematical framework that specifies a maximal amount of deviation to model outputs due to the presence of absence of any single training record. In the setting of Naive Bayes models, "Differentially Private Naive Bayes Classification" by J. Vaidya et al., Proceedings of IEEE WI/IAT, 2013, proposes to adapt a Naive Bayes classifier by adding Laplacian noise of the appropriate scale to its parameters. The computed parameters are then used to classify a new instance in the standard Naive Bayes fashion. Accordingly, due to the added noise, model outputs can be made to a large degree independent from a single training record.

### SUMMARY OF THE INVENTION

Various embodiments relate to classifiers that classify input instances into multiple classes based on multiple continuous probability distributions of respective features of the input instance and based on prior probabilities of the multiple classes. These classifiers typically also assume independence among the features. Such classifiers may be referred to broadly as Naive Bayes-type classifiers.

Although noise may be added to parameters of such classifiers to make the classifier outputs more or less independent from a single training record, doing so provides only statistical guarantees. Moreover, adding noise necessarily decreases the accuracy of the classification outputs, in some cases greatly decreasing the value of the model. Also, the approach of Vaidya et al. and the framework of differential privacy more generally concerns the influence of single records on model outputs, and so may not be able to sufficiently limit the dependence of model outputs on multiple training records. Fundamentally, the more records the model would need to be made more independent of, the more noise would need to be added and thus the more accuracy would have to be sacrificed. Effectively, adding noise provides a trade-off in which making parameters, and accordingly also classification outputs, more independent from training records results in a lower accuracy of the classification outputs that are obtained. For example, in various situations, applying noise to model parameters may not be regarded as a sufficient measure to satisfy right-to-be-forgotten requests arising due to the GDPR and similar other privacy regulations.

In accordance with a first aspect of the invention, a system for processing a classifier is proposed, as defined by claim 1. In accordance with another aspect of the invention, a corresponding computer-implemented method is proposed, as defined by claim 13. In accordance with an aspect of the invention, a computer-readable medium is provided as defined by claim 15.

In various embodiments, advantageously, to perform classification, a Naive Bayes-type classifier operating on continuous features may be used. This classifier may be made independent from one or more undesired training instances after the model has been trained and preferably also after the model has been deployed. For example, the model as deployed may initially depend on the one or more undesired training instances and, upon receiving a removal request message indicating the one or more undesired training instances, may be made independent of those training instances. By acting upon receiving a removal request message, interestingly, the model can be made independent from one or more *specific* training instances instead of having to make the model independent from any one training instance without knowing which. This way, for example, adding large amounts of noise to model outputs may be avoided.

Interestingly, the inventors envisaged that such a Naïve Bayes classifier can be made independent of undesired training instances in an operation that uses the parameters of the classifier and the undesired training instance, but that does not necessarily require access to the original training dataset on which the classifier has been trained. Namely, as the inventors realized, for a Naïve Bayes classifier, adapted parameters of a continuous probability distribution of an input feature may be computed based on current parameters of this probability distribution and the one or more undesired training instances. Moreover, an adapted prior probability of a class may be computed based on a current prior probability of the class and the one or more undesired training instances. Both may be peformed without accessing the original trianing dataset on which the model wa trained. Making a model independent from undesired training instances may be referred to generally as "detraining" the model with respect to undesired training instances.

In various embodiments, interestingly, a system dealing with removal request messages may access the classifier, but in many embodiments does not require access to the training dataset. For example, the training dataset may be deleted after the training of the classifier is completed. Accordingly, the amount of personal or otherwise sensitive information that needs to be stored may be limited.

By making the classifier independent from specific training instances and by doing so only upon receiving a removal request message for those specific instances, it may be enabled to still use the training instances while possible, e.g., while a data subject has not withdrawn consent. Moreover, by making the classifier independent from specific training instances, for example, it may be avoided to add generic noise that is large enough to hide any particular training instance. In fact, the adapted parameters of the classifier may be optimal with respect to the available records of the training dataset, e.g., both before and after dealing with the removal request message, the classifier may be able to provide classifier outputs with maximal accuracy given the records that the classifier output may be based on. For example, the adapted classifier may correspond to a classifier that obtained by training on a remainder dataset from which the undesired training instances are removed.

By adapting the parameters of the probability distributions and the prior probabilities with respect to the undesired training instances, interestingly, an adapted classifier may be obtained that is independent from the one or more undesired training instances in the sense that the parameters of the adapted classifier may be obtained by training on a dataset that is independent from the one or more undesired training instances, e.g., the set of parameters may also be obtainable by training a classifier from scratch based on the remainder dataset obtained by removing the undesired training instances from the training dataset. In that sense, the one or more undesired training instances may be regarded as being completely removed from the trained classifier. Accordingly, after dealing with the removal request, the undesired training records may be considered to be erased from the trained classifier and from the classifier outputs resulting from applying the trained classifier. In fact, for various Naïve Bayes-type classifiers, the optimal set of parameters may be computable as a deterministic function of the training dataset, and the updating may allow to exactly recover the optimum after removing the undesired training instances. In that case, the adapted model may even be equal to a model trained on the remainder dataset.

It is noted that the adaptation of the trained classifier does not need to be performed by the same parties that apply the classifier to input instances, and in particular, not all parties that use the classifier may need to receive the undesired training instances. For example, a system may be configured to deal with removal request messages and, having determined an adapted classifier in response to one or more removal request messages, provide the adapted classifier to one or more other systems for applying the classifier to input instances. In such cases, although the system dealing with the removal request messages may obtain the undesired training instances, systems that obtain the adapted model and apply it may not need such access. Accordingly, the exposure of sensitive information may be limited, further improving security.

Interestingly, by using the current parameters and current prior probabilities to determine adapted parameters and prior probabilities, removal request messages may be dealt with efficiently. For example, a full re-training of the classifier based on a remainder dataset may be avoided. As the inventors realized, since in a Naïve Bayes-type classifier, different features and different classes may each have their own respective set of parameters, also the updating of the parameters may be performed efficiently on a feature-by-feature and/or class-by-class basis. In particular, parameters of the continuous probability distributions of the respective features may be adapted on a parameter-by-parameter basis, a parameter being updated based on the current value of the parameter and possibly other parameters of the continuous probability distribution, and on the undesired training instances. Examples of such continuous probability distributions are provided throughout. Moreover, generally, continuous probability distribution parameters and prior probabilities may be updated with respect to any number of undesired training instances, e.g., at most or at least 1% of the training dataset, at most or at least 10% of the training dataset, or at most or at least 50% of the training dataset.

Generally, a removal request message may be sent for various reasons. For example, a removal request message may represent an absence of consent to further use a training instance, e.g., a withdrawal of consent to use the training instance. This can be the case when the training instance comprises personal information about a certain user. For example, the user itself may send the withdrawal of consent. Such a withdrawal of consent is sometimes also known as a right-to-be-forgotten request or right-to-erasure request. The withdrawal of consent can also be automatic, for example, the user may have provided a conditional consent, e.g., a time-limited consent or a consent dependent on another type of condition, and/or consent may be withdrawn by another party than the user: for example, another party with which a data sharing contract is in place. In these and other cases, the removal request message may be received from a consent management system configured to send the removal request message upon detecting that consent for using a training instance from the training dataset is missing. Such a consent management system can be combined with the system for processing a model, for example, in a single device.

The removal request message does not need to represent an absence of consent to further use the training instance, however. For example, it may be detected, e.g., in an anomaly detection system, that a training instance represents an adversarial instance, sometimes also called poisonous instance. For example, another party may have provided the instance to manipulate the classifier, e.g., to maliciously sway its decision boundary. Also in such cases, it is desirable to make the classifier independent of such adversarial instances. An instance may also be determined to be outdated, for example. In such cases, by making the classifier independent of undesired training instances, accuracy of the classifier may be improved. The classifier may also be made independent from one or more training instances to enable a deployment of the classifier at a different site, e.g., in a different country. For example, for one or more training instances, no consent for processing at the different site may be available, or it may be desired to roll out different versions at different sites, e.g., a free version vs a paid version, etcetera. In such cases, adapted classifiers for respective sites may be determined and provided to one or more respective sites.

The techniques described herein are applicable to various kinds of data, in particular sensor data such as audio data, image data, video data, radar data, LiDAR data, ultrasonic data, motion data, thermal imaging data, or various individual sensor readings or their histories. For example, in various embodiments, sensor measurements may be obtained from one or more sensors via a sensor interface, e.g., from a camera, radar, LiDAR, ultrasonic, motion, or thermal sensors, or various sensors for measuring physiological parameters such as heart beat or blood pressure, or any combination. For example, an instance may comprise time series data of one or more of such sensors. Based on these sensor measurements, an input instance may be determined to which the classifier is applied. Naïve Bayes-type classifiers may be particularly effective in cases with a relatively large amount of features, for example, in which the number of features is within an order of magnitude of the number of training instances on which the model is trained, or in which the number of features is at least 50 or at least 100.

Apart from the embodiments illustrated throughout, various additional embodiments are also envisaged in which the techniques for processing a classifier as described herein may be advantageously applied.

In an embodiment, the classifier may be applied in a control system for controlling a computer-controlled machine, e.g., a robot, a vehicle, a domestic appliance, a power tool, a manufacturing machine, a personal assistant, an access control system, etc. The control system may be part of or separate from the computer-controlled machine. For example, a control signal may be determined by the control system based at least in part on a classification by the classifier. As input, the classifier may obtain data indicative of a state of the computer-controlled machine and/or the physical environment it operates in.

The classifier may also be applied in various systems for conveying information, e.g., a surveillance system based on images of a building or other object under surveillance, or a medical imaging system, e.g., based on an image of a body or part of it. The classifier may also be used, for example, in an optical quality inspection system for manufacturing process to inspect manufactured objects for failures. For example, a classification into failure/non-failure and/or into particular failure types may be made from images of the manufactured objects.

In an embodiment, the classifier may be applied in an autonomous vehicle. For example, an input instance may comprise an image of the environment of the vehicle. The model can for be for classifying traffic signs, pedestrian behaviours, road surfaces, other vehicles, etc. In various cases, a classifier output may be used at least in part to control the autonomous vehicle, for example, to operate the autonomous vehicle in a safe mode upon detecting an anomaly, e.g., a pedestrian unexpectedly crossing the road.

In an embodiment, the classifier may be applied in medical image classification. For example, the model may be used to detect a tumour or other object of medical relevance in an image, e.g., a MRI, CT, or PET scan, of a body or part of it, or the model may be used to classify images into different pathologies or other types of medical outcomes.

In an embodiment, the classifier may be applied for signal processing of measurements of various external devices, e.g., loT devices. For example, the classifier may be applied to a streams of incoming sensor measurements of a device, for example, to detect anomalies or other types of events.

In an embodiment, the classifier may be applied for predictive maintenance, for example to predict whether a component, e.g., a screen or a battery, of a larger device, e.g., a car or a medical device, needs to be replaced based on usage data, e.g., time-series data.

In an embodiment, the classifier may be used in a system for training an autonomous device such as a robot to interact in a physical environment, for example, in a model used to determine an input to a reinforcement learning system, e.g., by imitation learning. For example, classifications provided by the classifier may be used as input features for a reinforcement learning system.

Optionally, the removal request message may include the one or more undesired training instances, for example, in the form of features to which the classifier may be applied. By receiving the undesired training instances as part of the removal request message, it may be avoided to have to retrieve the undesired training instances from the training dataset itself. For example, in some embodiments no access to the training dataset may be needed, reducing the need to store sensitive information. In other embodiments, however, at least some information about the training dataset may still be used, e.g., accessed locally or queried at an external location, to check whether the undesired training instances are actually part of the training dataset, and/or to keep track of which training instances of the training dataset have been identified as being undesired.

Optionally, having obtained undesired training instances, it may be checked if an undesired training instance is present in the training dataset by computing a hash of the undesired training instance and checking said presence based on the hash. Various ways of checking presence based on a hash may be envisaged. For example, hashes of respective training instances of the training dataset may be stored as a list, by storing counters for respective hash values, or represented in compressed form by a Counting Bloom filter. Along with modifying the classifier, also the stored representation of the hashes of the training instances may be updated, e.g., by removing the hash from the list, decreasing a counter, or updating the counting Bloom filter. By checking that an undesired training instance is present, it may be avoided to inadvertently remove a training instance multiple times or to allow adversarial influencing of the classifier through the provision of undesired training instances that were not originally part of the training dataset of the classifier.

Optionally, the hash may be a seeded hash, e.g., with the seed being determined upon inclusion of the training instance in the training dataset, e.g., by a party providing the training instance or a party training the model. Seeds are also commonly known in the art as "salts". For example, the seed may be determined by or sent to a user whose personal information is included in the model, the seed effectively providing a way for the user to prove that their information was included in the model and preventing others from removing their information. Additionally, using a seeded hash may improve privacy by making it hard or even impossible to check, based on the stored hashes of the training instances, whether a given training instance is present in the training dataset. For example, by including the seed, it may not be possible to go through potential training instances one-by-one, hash them, and verify whether they are comprised in the training dataset based on the stored hashes, since such a check would require to know the seed. For example, the seed may comprise at least 10, 20, or 40 bits of entropy.

Optionally, the one or more undesired training instances identified in the removal request message may be retrieved from a training dataset on which the classifier has been trained. Along with adapting the classifier, also the training dataset may be updated by removing the one or more undesired training instances from the training dataset. In such cases, it is not necessary for the removal request messages to comprise the training instances themselves, e.g., users do not need to store training instances relating to them to be able to send removal request messages. Although the party updating the classifier may in this case need access to the training dataset, still, this access may only be needed to handle removal request messages and can accordingly be made more restricted than access to the parameters of the classifier itself, e.g., using additional logging or access control measures.

Optionally, the training dataset may comprise multiple training instances collected from respective users. Accordingly, the training instances may represent personal information about these users. The removal request message may indicate a user whose training instances are to be removed from the training dataset. For example, records may be stored along with an associated user identifier, the removal request message specifying the user identifier. A removal request message can also indicate the user by specifying the particular records of the user to be removed. Enabling to remove data associated with a particular user may allow to deal appropriately with right-to-erasure requests, also known as right-to-be-forgotten-requests, and/or with users withdrawing consent. A removal request message may indicate data of multiple users.

Optionally, the processing of a classifier may be combined with performing anomaly detection. For example, it may be detected that at least one training instance represents an adversarial instance, using techniques known per se, and based on such a detection, the removal request message for said training instance may be sent, e.g., via internal communication, for it to be retrieved and dealt with as described herein. Accordingly, if it is detected that a training instance represents an adversarial instance, the classifier may be made independent from the training instance and accordingly the adversarial influencing by the instance may be prevented in the adapted model.

Optionally, a training instance of a user may comprise one or more sensor measurements of the user. For example, a measurement may be an image of the user, a measurement of a physiological quantity of the user such as a blood pressure or heart rate, etcetera. The measurement can also be a genomic sequence of the user, a fingerprint, and the like. The data may be measured using any appropriate sensor. Since such measured data is intrinsically related to the user, it may be particularly privacy-sensitive and accordingly, being able to remove training instances with such data from a dataset may be particularly desirable.

Optionally, a training instance of a user may be collected by receiving the training instance from a user device. Such a training instance may comprise a sensor measurement by the user device of a physiological quantity of the user, such as a heart rate and/or a blood pressure. For example, the user device may be a smart watch, smart phone, or other kind of wearable device, a home medical measurement device, or the like. The user device may provide the training instance as an instance for which a classifier output is desired. For example, upon receiving an instance from the user device, the classifier may be applied to the instance and a classifier output provided to the user device, the instance being used at a later stage as a training instance to refine the model. Aside from the training instance, also the removal request message may be received from the user device itself, for example, the user may change a setting on the user device to withdraw consent for processing of the measurements of the user device. The removal request message may also be sent by the user from another device, however, e.g., by logging into a user account also used by the user device.

Optionally, the classifier may comprise a feature extractor for determining the features of the input instance from the input instance. Although Naïve Bayes-type classifiers are typically relatively simple, by including a feature extractor, still, more complicated machine learning tasks such as image classification may be performed. For example, the VGG net trained by the Oxford Visual Geometry Group is used in practice as a feature extractor for various applications. In such cases, the feature extractor and the rest of the classifier may be trained on different datasets, the feature extractor being trained on a further dataset not comprising the undesired training instance. For example, the feature extractor may be a pre-trained feature extractor, e.g., trained on a relatively large dataset, the classifier being obtained by taking the pre-trained feature extractor and just training the classifier on the extracted features. The feature extractor may be trained by a third party or even be offered as a service to the party applying the classifier, e.g., as part of the AI platforms of Google and Microsoft, and the like. The feature extractor is typically not a Naïve Bayes classifier, e.g., it can be a neural network, e.g., a convolutional network. For example, the feature extractor can be an encoder part of an autoencoder, or the like.

Accordingly, the classifier may be adapted by adapting the parameters of the part of the model performing the classification, but not the parameters of the feature extractor. The use of a separate feature extractor may be beneficial because of expressiveness of the model, e.g., because a feature extractor may be used that can relatively complex and/or can be optimized, e.g., trained on a relatively large dataset, and shared among multiple classifiers or other models. Apart from this, using a feature extractor in combination with a Naïve Bayes-type model may be especially beneficial for allowing to relatively easily update the classifier to remove undesired training instances. For example, compared to other types of classifier, fewer parameters may need to be updated and only a part of the model may need to be re-trained, improving efficiency.

Optionally, a continuous probability distribution may be parametrized by one or more moments of the continuous probability distribution, e.g., comprising one or more of a mean, a variance, a skewness, and a kurtosis. For example, normal distributions such as a univariate or multivariate normal distribution or a matrix normal distribution may be defined by their means and variances, covariances, and/or standard deviations. However, also other types of continuous probability distributions may be defined in terms of their moments at least in the sense that their probability density function in a given point may be computed from the moments. For example, in some cases, their "regular" parameters may be computed from the moments. For example, in the case of an exponential distribution, its rate parameter may be computed as 1/*µ* where *µ* is the mean. In such cases, the parameters of the probability distribution may be updated in terms of their moments and used, e.g., to evaluate the probability density function as usual. For example, the moments may be included as parameters of the continuous probability distribution instead of or in addition to their original parameters, e.g., an exponential distribution may be parametrized by its mean, in which case its rate may not need to be stored in addition. Interestingly, the moments of a probability distribution may be updated based on their current values and the undesired training instances relatively efficiently, e.g., a mean may be adapted using the current mean and the one or more undesired training instances, and a variance may be updated using the current mean and variance and the one or more undesired training instances, as further discussed elsewhere.

Optionally, a prior probability of a class may be adapted based on the current prior probability of the class and the one or more undesired training instances. Also in this case, access to the training dataset may not be needed and the adaptation can be performed efficiently.

Optionally, following the adaptation of the classifier, a query instance may be obtained and the adapted classifier may be applied to the query instance to obtain a classifier output independent from the one or more undesired training instances. As also discussed elsewhere, the adaptation of the classifier and the application of the classifier to query instances may be performed by the same system or different systems. It is also possible for both the adapting and/or the applying to be performed multiple times, for example, in an interleaved fashion in which, at some point after an adapting, an applying is performed, and at some point after the applying, another adapting is performed, etcetera. For example, a system may be configured to obtain multiple respective removal request messages and/or model application messages and to respond to these messages accordingly by adapting or applying the classifier. Optionally, the party determining the adapted classifier may have previously trained the model on the training dataset. In such cases, the party may store the training dataset or a representation of hashes of training instances for use in processing removal request messages, as also discussed elsewhere. Accordingly, potentially sensitive information in the training dataset may be kept local to the party performing the training and/or adaptation, for example, whereas the original trained classifier and its adaptations may be provided to other parties for application to query instances.

Optionally, multiple removal request messages may be received and dealt with in a single operation of making the classifier independent. For example, multiple removal request messages may be collected, e.g., until a certain, preferably rather short, time window has passed, e.g., of at most a minute or at most thirty minutes. Instead or in addition, multiple removal request messages may be collected until a certain maximum amount of messages has been received and/or time has passed to ensure that use of the undesired training instances is avoided as much as possible. It is also possible, instead or in addition, to deal with any pending removal request messages when a new query instance arrives. By accordingly batching multiple removal request messages, efficiency is improved, while still avoiding that training instances affect classification outputs too much.

It will be appreciated by those skilled in the art that two or more of the above-mentioned embodiments, implementations, and/or optional aspects of the invention may be combined in any way deemed useful.

Modifications and variations of any system and/or any computer readable medium, which correspond to the described modifications and variations of a corresponding computer-implemented method, can be carried out by a person skilled in the art on the basis of the present description, and similarly, for modifications and variations of a method or medium based on described modifications and variations of a system.

### BRIEF DESCRIPTION OF THE DRAWINGS

These and other aspects of the invention will be apparent from and elucidated further with reference to the embodiments described by way of example in the following description and with reference to the accompanying drawings, in which:
Fig. 1 shows a system for processing a classifier;
Fig. 2 shows a detailed example of how to make a classifier independent from one or more undesired training instances, and how to apply the classifier to an input instance;
Fig. 3 shows a detailed example of how to make a classifier independent from one or more undesired training instances, where the classifier comprises a feature extractor;
Fig. 4 shows a computer-implemented method of processing a classifier;
Fig. 5 shows a computer-readable medium comprising data.

It should be noted that the figures are purely diagrammatic and not drawn to scale. In the figures, elements which correspond to elements already described may have the same reference numerals.

### DETAILED DESCRIPTION OF EMBODIMENTS

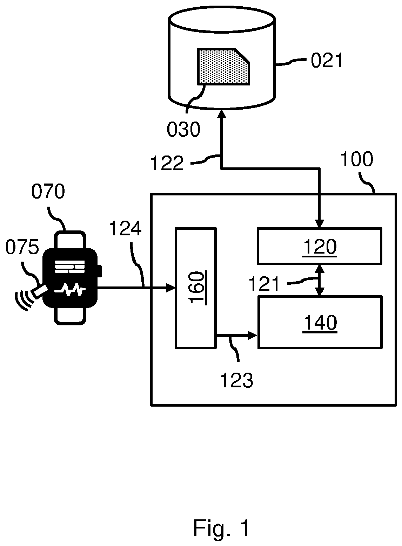

**Fig. 1** shows a system 100 for processing a classifier. The classifier may classify an input instance into multiple classes based on multiple continuous probability distributions of respective features of the input instance and based on prior probabilities of the multiple classes. The system 100 may comprise a data interface 120 and a processor subsystem 140 which may internally communicate via data communication 121. Data interface 120 may be for accessing the model 030. In various embodiments, the data interface 120 may also be for accessing the training dataset on which the model has been trained or a representation of hashes of training instances of the training dataset.

The processor subsystem 140 may be configured to, during operation of the system 100 and using the data interface 120, access data 030 representing the classifier. For example, as shown in Fig. 1, the data interface 120 may provide access 122 to an external data storage 021 which may comprise said data 030. Alternatively, the data 030 may be accessed from an internal data storage which is part of the system 100. Alternatively, the data 030 may be received via a network from another entity. In general, the data interface 120 may take various forms, such as a network interface to a local or wide area network, e.g., the Internet, a storage interface to an internal or external data storage, etc. The data storage 021 may take any known and suitable form.

System 100 may also comprise a removal request interface 160 configured for receiving a removal request message 124. The removal request message 124 may identify one or more undesired training instances of the training dataset. Removal request interface 160 may internally communicate with processor subsystem 140 via data communication 123. Removal request interface 160 may be arranged for direct communication with other systems from which removal request messages may be received, e.g., user devices, e.g., using USB, IEEE 1394, or similar interfaces. Removal request interface 160 may also communicate over a computer network, for example, a wireless personal area network, an internet, an intranet, a LAN, a WLAN, etc. For instance, removal request interface 160 may comprise a connector, e.g., a wireless connector, an Ethernet connector, a Wi-Fi, 4G or 4G antenna, a ZigBee chip, etc., as appropriate for the computer network. The figure shows a removal request message 124 being received from smart watch 070, for example via the internet, where the smart watch 070 is also configured to measure one or more physiological quantities of the user using one or more sensors, such as sensor 075 shown in the figure. System 100 may form a user data processing system together with one or more user devices 070 and/or other systems that apply the model.

Removal request interface 160 may also be an internal communication interface, e.g., a bus, an API, a storage interface, etc. For example, system 100 may be part of a consent management system configured to ensure that consent is available for the training dataset; for example, another part of the consent management system may send a removal request message to system 100 as described herein. As another example, system 100 may be part of an anomaly detection system configured to detect and deal with undesired training instances, e.g., adversarial examples or other types of outliers, in which case another part of the anomaly detection system may send a removal request message to system 100 as described herein.

Processor subsystem 140 may be configured to, during operation of the system 100 and using the data interface 120, upon receiving the removal request message 124, make the classifier independent from the one or more undesired training instances. To make the classifier independent, processor subsystem 140 may be configured to, for a continuous probability distribution of a feature, computing adapted parameters of said probability distribution based on current parameters of said probability distribution and the one or more undesired training instances. Further, processor subsystem 140 may compute an adapted prior probability of a class based on a current prior probability of the class and the one or more undesired training instances. Accordingly, an adapted classifier may be obtained.

As an optional component, the system 100 may comprise an image input interface or any other type of input interface (not shown) for obtaining sensor data from a sensor, such as a camera. Processor subsystem 140 may be configured to obtain an input instance for the classifier based on the obtained sensor data, and to apply the adapted classifier to the obtained input instance. For example, the camera may be configured to capture image data, processor subsystem 140 being configured to determine an input instance from the image data. The input interface may be configured for various types of sensor signals, e.g., video signals, radar/LiDAR signals, ultrasonic signals, etc. As an optional component, the system 100 may also comprise a display output interface or any other type of output interface (not shown) for outputting a classifier output of the adapted model for an input instance to a rendering device, such as a display. For example, the display output interface may generate display data for the display which causes the display to render the classifier output in a sensory perceptible manner, e.g., as an on-screen visualisation, e.g., alongside the input instance. As an optional component, the system 100 may also comprise an actuator interface (not shown) for providing, to an actuator, actuator data causing the actuator to effect an action in an environment of system based on a classifier output determined for an input instance.

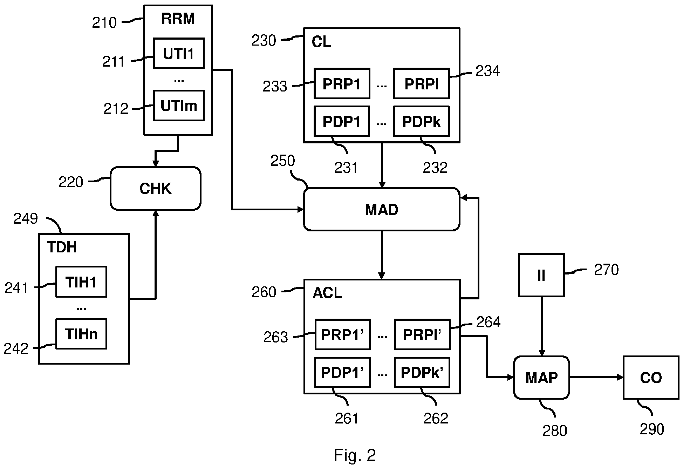

Various details and aspects of the operation of the system 100 will be further elucidated with reference to Figs. 2-3, including optional aspects thereof.

In general, the system 100 may be embodied as, or in, a single device or apparatus, such as a workstation, e.g., laptop or desktop-based, or a server. The device or apparatus may comprise one or more microprocessors which execute appropriate software. For example, the processor subsystem may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the functional units of the system, e.g., the data interface and the processor subsystem, may be implemented in the device or apparatus in the form of programmable logic, e.g., as a Field-Programmable Gate Array (FPGA) and/or a Graphics Processing Unit (GPU). In general, each functional unit of the system may be implemented in the form of a circuit. It is noted that the system 100 may also be implemented in a distributed manner, e.g., involving different devices or apparatuses, such as distributed servers, e.g., in the form of cloud computing.

**Fig. 2** shows a detailed yet non-limiting example of how to process a classifier to make the classifier independent of one or more undesired training instances, and how to apply the accordingly adapted classifier to an input instance.

Shown in the figure is a Naïve Bayes-type classifier **CL**, 230. The classifier may be configured to classify an input instance into multiple classes, e.g., to assign a class of the multiple classes to an input instance. The classifier **CL** can be a binary classifier, or a multiclass classifier, for example, with at least three, at least five, or at least ten classes. Classifier **CL** may classify an input instance into the multiple classes based on multiple continuous probability distributions of respective features of the input instance and based on prior probabilities of the multiple classes. The figure shows classifier **CL** comprising prior probabilities **PRP1**, 233, up to **PRPI**, 234, and probability distribution parameters **PDP1**, 231, up to **PDPk**, 232.

Specifically, classifier **CL** may comprise parameters **PDP*** of class-conditional continuous probability distributions for respective features of an input instance. To classify an input instance, respective probabilities of the input instance belonging to respective classes may be determined based on the respective prior probabilities **PRP*** of the classes and class conditional probabilities of features of the input instance occurring in that respective class according to the corresponding continuous probability distributions **PDP***. Mathematically, for example, the following decision function can be used: 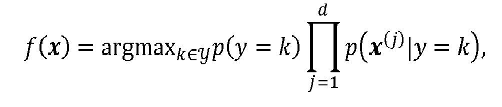 where ***x***^{(*j*)} is the *j*th feature value of an input instance, e.g., ***x*** = [***x***⁽¹⁾, ***x***⁽²⁾, ***x***^{(d)}]*^{T}*.

Typically, the prior probabilities **PRP***, e.g., *p*(*y* = *k*) and/or the parameters of the continuous probability distributions **PDP***, e.g., *p*(***x***^{(*j*)}|*y* = *k*), may be obtained by training the classifier on a training dataset, in other words, by estimating the parameters from the training dataset. Generally, the training dataset may comprise one or more sensor measurements of a user, for example, an image represented by pixels, features, or the like, or measurements of various physiological quantities, e.g., in a time series, etcetera. Training of classifier **CL** on training dataset, in this case in a supervised learning setting, may be formulated as follows. A training dataset may be denoted ${D}_{train} {= \left\{{u}_{i}\right\}}_{i = 1}^{N} ,$ where a training instance may be denoted *uᵢ* = (***x**ᵢ*,*yᵢ*). For example, an input instance may comprise an input feature vector ***x**ᵢ* ∈ χ, e.g., 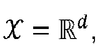 and a target value *yᵢ* ∈ *Y*. For example, *Y* may equal {0,1, ..., *C* - 1} with C the total number of classes. Generally, classifier **CL** may be trained on the training dataset to learn a function *f*: *X* → *Y* that generalizes from the training dataset to unseen input instances.

In the case of Naïve Bayes-type models, the classifier **CL** may be trained by determining parameters of respective continuous distributions of respective features of input instance based on instances of the training dataset. Generally, a continuous probability distribution may be parameterized by one or more of its moments. For example, one way of modelling a class conditional probability for a feature is by using the Gaussian distribution based on its first and second moments, e.g.: $p \left(x=a|y=k\right) = \frac{1}{\sqrt{2 {πσ}_{k}^{2}}} exp \left(-\frac{{\left(a-{μ}_{k}\right)}^{2}}{2 {σ}_{k}^{2}}\right) ,$ where µ*ₖ* and ${σ}_{k}^{2}$ are the first moment, e.g., the class conditional feature mean, and the second moment, e.g., the class conditional feature variance, respectively, of the continuous probability distribution. For example, the mean and variance may form the probability distribution parameters **PRP*** for this feature. For simplicity, in the above formula, the feature superscript (*j*) is dropped, and *x* is used to denote the values of the *jth* feature of ***x***. Apart from the Gaussian distribution, also various other continuous probability distributions may be used, e.g., an exponential distribution, etc.

As highlighted in the above example, typically, respective features of the input instance are real numbers, and accordingly, the respective probability distributions may be univariate. This is not necessary however, e.g., one or more features may be vectors of real numbers, modelled by respective multivariate probability distributions. However, also in this case there may be multiple such features, and the vectors modelled by a multivariate probability distribution may comprise only few elements, e.g., two, three, at most five, or at most ten. The number of parameters of a probability distribution **PDP*** may also be limited, for example, one, more, or or all of the continuous probability distributions may be modelled by one, two, at most three, or at most five, parameters **PDP***.

It is noted that, in order to apply classification model **CL** to an input instance, it is typically not needed to access the training dataset. For example, respective probabilities of an input instance belonging to respective classes may be determined based on the prior probability **PRP*** of that class and parameters of a class-conditional probability distributions **PDP*** of features for that class.

Also shown in the figure is a removal request message **RRM**, 210. A removal request message may identify one or more undesired training instances **UTI1**, 211, up to **UTIm,** 212, of the training dataset on which classifier **CL** has been trained.

The undesired training instances **UTI*** may be indicated in various ways. In some embodiments, the undesired training instances **UTI*** are included in the removal request message itself. Interestingly, in this case, access to the training dataset may not be needed to make the classifier **CL** independent from the undesired training instances **UTI*.** Removal request message **RRM** may also indicate the undesired training instances **UTI*** in the training dataset, e.g., by including indices or other types of identifiers of the undesired training instances. For example, the training dataset may be obtained by collecting multiple training instances of respective users, in which case the removal request message **RRM** can indicate a user whose training instances are to be removed from the training dataset, e.g., by means of a user identifier. In such cases, the undesired training instances may be obtained by retrieving them from the training dataset. Along with making the classifier **CL** independent from the undesired training instances **UTI*,** the one or more undesired training instances may also be removed from the training dataset to make the training dataset independent from the undesired training instances as well.

In various embodiments, to ensure that only undesired training instances are dealt with that are actually included in the training dataset, e.g., to avoid processing undesired training instances that were already removed or that were never included in the training dataset to begin with, a checking operation **CHK,** 220, may be performed, Checking operation **CHK** may check if an undesired training instance **UTI*** is present in the training dataset. Such a check may be performed by accessing the training dataset, but interestingly, this is not needed.

Namely, in various embodiments, checking operation **CHK** may compute hashes of undesired training instances **UTI*** and use these hashes to check for presence in the training dataset. Accordingly, a representation of the hashes of the training instances of the training dataset may be accessed that allows for such a check to be performed. By using such hashes to perform the check, and storing a representation of such hashes to allow the check, storage of sensitive data may be reduced since the input instances may not be derivable from the hash. For example, the hash may be any one-way function, e.g., folding, division hashing, or a cryptographic hash function such as MD5 or SHA2. The hash may be salted, e.g., random salt data (not shown in the figure) may be included in the training input that is hashed, to make it even more difficult to recover training instances from the representations of their hashes. In this case, for example, the salt may be included in the removal request message **RRM.**

For example, as illustrated in the figure, a set **TDH**, 249, of training dataset hashes may be accessed. This set may comprise hashes of training instances of the training dataset on which the classifier **CL** has been trained. Shown are training instance hashes **TIH1**, 241, up to **TIHn**, 242. By checking whether the hash an undesired training instance is comprised in the set, it may be checked whether the undesired training instance is comprised in the training dataset. Along with adapting the classifier, also the set of hashes **TDH** may be updated to remove the undesired training instances that were removed from classifier **CL.** Instead of storing hashes individually, it is also possible, for example, to use a more compressed representation such as a counting Bloom filter. Also a counting Bloom filter may be updated to remove undesired training instances along with removing them from the classifier **CL** itself. Accordingly, storage may be reduced while still providing reasonably strong guarantees that attempts to remove instances that were not actually in the training dataset are detected.

Upon receiving the removal request message **RRM**, classifier **CL** may be made independent from the one or more undesired training instances **UTI*.** To this end, in a model adaptation operation **MAD**, 250, an adapted classifier **ACL**, 260 may be determined as a classifier for the remainder dataset obtained by removing the undesired training instances **UTI*** from the dataset on which classifier **CL** was trained. Typically, the adapted classifier **ACL** has the same structure as the original classifier **CL**, e.g., the same function or procedure may be used to determine the classifier output in the adapted classifier **ACL** as in the classifier **CL**, but based on a different set of parameters. Accordingly, as shown in the figure, also the adapted classifier **ACL** may be parametrized by parameters **PDP1'**, 261, up to **PDPk'**, 262, of multiple continuous probability distributions of respective features of an input instance, e.g., class-conditional probability distributions. Moreover, as shown, the adapted classifier **ACL** may be parameterized by prior probabilities **PRP1'**, 263, up to **PRPI'**, 264, of the respective classes into which the adapted classifier **ACL** can classify.

Interestingly, as also discussed above, a Naïve Bayes classifier **CL** may classify instances by making separate use of class prior probabilities and probability distributions of features, e.g., of class-conditional probabilities. For example, this can be seen from the example decision function discussed above: 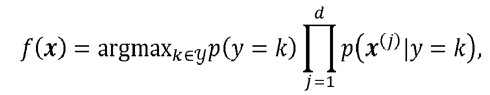 As a consequence of this model structure, also the classifier **CL** can be made independent from undesired training instances by separately adapting parameters of the respective parts of the classifier **CL.** Accordingly, irrespective of how exactly the update is performed, already because of this structure of the classifier the update can be relatively efficient.

Specifically, as part of model adaptation operation **MAD**, for a continuous probability distribution of a feature, adapted parameters **PDP***' of this probability distribution may be computed based on current parameters **PDP*** of this probability distribution and the one or more undesired training instances **UTI***. Specifically, in case the probability distribution comprises moments, these moments can be separately updated. For example, a Gaussian distribution defined by a mean and variance can be adapted by adjusting the mean and variance.

For example, the mean included in parameters **PDP***' of a probability distribution may be adapted using the current mean included in corresponding parameters **PDP*** and the one or more undesired training instances **UTI***. Mathematically, one may denote the original mean and variance by µ and *σ*². Then µ' and *σ*'² may be used to denote the updated mean and variance after removing one or more undesired training instances. For example, for the case of a single undesired training instance *u'* = (*x', y'*), the updated mean may be computed as derived as follows: $\begin{matrix}µ = \frac{1}{N} {\sum }_{{D}_{train}} x & \left(mean of the whole dataset, including xʹ\right) \\ = \frac{1}{N} \left[xʹ+{\sum }_{{D}_{trainʹ}} x\right] & \left(decomposing the sum\right) \\ = \frac{xʹ}{N} + \frac{c}{N} & \left(where C={\sum }_{{D}_{trainʹ}} x\right) \\ = \frac{xʹ}{N} + \frac{1}{\frac{N - 1}{C} + \frac{1}{C}} & \\ = \frac{xʹ}{N} + \frac{1}{\frac{1}{µʹ} + \frac{1}{C}} & \left(sinc by definition µʹ=\frac{C}{N - 1}\right)\end{matrix}$ $μʹ = {\left[\frac{N}{Nμ - xʹ}-\frac{1}{C}\right]}^{- 1}$ (by rearranging the terms)

Similarly, the variance may be adapted using the current mean and variance and the one or more undesired training instances, e.g., for a single undesired training instance, the following formula may be derived: $\begin{matrix}{σ}^{2} & = \frac{1}{N} {\sum }_{{D}_{train}} {\left(x-μ\right)}^{2} & \left(variance of the whole dataset, including xʹ\right) \\ & = \frac{1}{N} \left[{\left(xʹ-μ\right)}^{2}+{\sum }_{{D}_{trainʹ}} {\left(x-μ\right)}^{2}\right] & \\ & = \frac{1}{N} \left[{\left(xʹ-μ\right)}^{2}+{\sum }_{{D}_{trainʹ}} {\left(x-μʹ+Δμ\right)}^{2}\right] & \left(where Δμ=μʹ-μ\right) \\ & = \frac{1}{N} \left[{\left(xʹ-μ\right)}^{2}+{\sum }_{{D}_{trainʹ}} {\left({Δ μ}^{2}+Δμ\left(x-μʹ\right)\right)}^{2}\right] + \frac{1}{N} {\sum }_{{D}_{trainʹ}} {\left(x-μʹ\right)}^{2} & \\ & = \frac{{C}_{1}}{N} + \frac{1}{N} \left[{\sum }_{{D}_{trainʹ}} {\left(x-μʹ\right)}^{2}\right] & ( {where C}_{1} = {\left(xʹ-μ\right)}^{2} + {\sum }_{{D}_{train}} \left[{Δ μ}^{2}+2Δμ\left(x-μʹ\right)\right] \\ & = \frac{{C}_{1}}{N} + \frac{{C}_{2}}{N} & \left({where C}_{2}={\sum }_{{D}_{trainʹ}} {\left(x-μʹ\right)}^{2}\right) \\ & = \frac{{C}_{1}}{N} + \frac{1}{\frac{N - 1}{{C}_{2}} + \frac{1}{{C}_{2}}} & \\ & = \frac{{C}_{1}}{N} + \frac{1}{\frac{1}{{σʹ}^{2}} + \frac{1}{{C}_{2}}} & \left({since σʹ}^{2}=\frac{{C}_{2}}{N - 1}\right)\end{matrix}$ ${σʹ}^{2} = {\left[\frac{N}{{Nσ}^{2} - {C}_{1}}-\frac{1}{{C}_{2}}\right]}^{- 1}$ (by rearranging terms)

Moreover, as part of the model adaptation operation **MAD**, an adapted prior probability **PRP*'** of a class may be computed based on a current prior probability **PRP*** of the class and the one or more undesired training instances **UTI***. Denote the updated prior probability for a class *k* after removing a training instance *u'* as *p'*(*y* = *k*)*.* Analogously to the above derivations, mathematically, the formula below can for example be used to compute the adapted prior probability of removing one undesired training instance: $pʹ \left(y=k\right) = {\left[\frac{N}{p \left(y=k\right) N - I \left(yʹ=k\right)}-\frac{1}{C}\right]}^{- 1} ,$ where $C = {\sum }_{{D}_{train}^{ʹ}} I \left(y=k\right) .$

In general, when adapting a probability distribution parameter or prior probability, multiple undesired training instances **UTI*** may be handled in a single computation, e.g., by appropriately generalizing the above formulas for mean, variance, and prior probability to the case of multiple undesired training instances, by applying the computation for a single undesired training instance multiple times.

Having determined adapted classifier **ACL**, as shown in the figure, a model application operation **MAP**, 280, may be used to apply the adapted classifier **ACL** to an input instance **II**, 270, resulting in a classifier output **CO**, 290. For example, model application **MAP** may be performed by the same system that determined the adapted classifier or by another system that obtains the adapted classifier. Interestingly, the classifier output **CO** may be considered to be independent of the undesired training instances **UTI*** at least in the sense that its set of parameters **PAR***' may represent an optimal classifier with respect to a remainder dataset from which the undesired training instances **UTI*** have been removed. Moreover, also the remainder dataset itself and the adapted classifier **ACL** may in that sense be considered independent of the undesired training instances **UTI***. Accordingly, an appropriate way of dealing with removal request message **RRM** is shown.

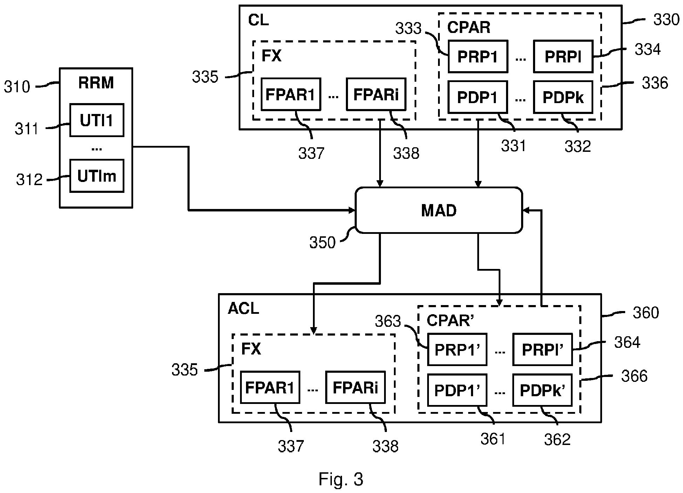

**Fig. 3** shows a detailed, yet non-limiting, example of how to process a Naïve Bayes-type classifier that uses a feature extractor to determine features of an input instance. This example may be based on the example of Fig. 2. In this example, by using a feature extractor, the expressiveness of the model may be greatly increased. Interestingly, however, it may still be possible to deal with undesired training instances. Shown in the figure is a classifier **CL**, 330, configured to classify an input instance into multiple classes based on multiple continuous probability distributions of respective features of the input instance and based on prior probabilities of the multiple classes. The classifier **CL** may be parameterized by classifier parameters **CPAR**, 336, which may include parameters **PDP1**, 331, up to **PDPk**, 332, of the continuous probability distributions. The parameters **CPAR** may also include the prior probabilities **PRP1**, 333, up to **PRPI**, 334.

Similarly to Fig. 2, one or more training instances of the training dataset on which classifier **CL** has been trained may be identified as undesired training instances in a removal request message **RRM**, 310. For example, shown in the figure are undesired training instances **UTI1**, 331, up to **UTIm**, 312. Various alternatives discussed with respect to Fig. 2 for obtaining the undesired training instances **UTI***, e.g., from the message **RRM** itself or the training dataset, apply here as well. In this case, the undesired training instances may also be represented as features extracted by the feature extractor discussed below. Accordingly, a model adaptation operation **MAD**, 350, may be performed to determine an adapted classifier **ACL**, 360 independent from the one or more undesired training instances **UTI*.**

Interestingly, in the example shown in this figure, classifier **CL** may comprise a feature extractor **FX**, 335. As shown in the figure, the feature extractor may be parametrized by a set of parameters **FPAR1**, 337, up to **FPARi**, 338. Feature extractor **FX** may be for determining the features of an input instance to which the classifier is applied. Accordingly, classifier **CL** may be applied to a query instance by applying the feature extractor **FX** to the query instance to obtain a feature representation of the query instance, and applying the classification model **CL**, using classification parameters **CPAR**, to the feature representation to obtain a classification output.

The classifier **CL** may be trained, in other words classification parameters **CPAR** determined, on a training dataset (not shown) including the undesired training instances **UTI***, for example, by fitting continuous probability distributions for respective extracted features to the training dataset and/or determining prior probabilities of classes from classes of instances of the training dataset, as known per se.

Interestingly, however, the feature extractor **FX** may be trained on a further dataset (not shown) that does not include the undesired training instances. For example, the feature extractor may be a pre-trained feature extractor, for example, obtained from a third party. Although the feature extractor is illustrated in the figure as comprising its set of parameters **FPAR***, it will be understood that the feature extractor **FX** may be an external feature extractor, e.g., accessed via an API, e.g., of a machine learning framework such as the Google AI Platform or the Microsoft AI Platform. Generally, the feature extractor **FX** may be shared among multiple classifiers and other models. Also, the feature extractor **FX** may be trained on a relatively large dataset, for example, of publicly available data, whereas the classifier **CL** may be trained on a smaller dataset. For example, the feature extractor may be the VGG network of Oxford University or a similar general pre-trained model. Various ways of training feature extractors may be used that are known per se. The feature extractor is typically not a Naïve Bayes-type model itself, e.g., it can be a neural network such as a deep neural network or a convolutional neural network, etc.

Interestingly, by using a general feature extractor **FX** trained on a relatively large dataset, a smaller dataset may suffice for training the classifier **CL.** For example, the training dataset **TD** may comprise at most 100, at most 1000 or at most 10000 training instances. On the other hand, the training dataset of the feature extractor may comprise at least 100000 or at least 1000000 training instances, for example. Although using a relatively small dataset for training the classifier may be beneficial from a performance and data collection effort point of view, this may also make it particularly relevant to properly deal with removal request messages, e.g., since a single instance of the training dataset TD may have a relatively greater influence on the parameters **CPAR** and/or classifier outputs of the classifier **CL.**

When determining adapted classifier **ACL,** parameters **FPAR*** of the feature extractor **FX** may be kept unchanged. For example, as shown in the figure, adapted classifier **ACL** may comprise the same feature extractor **FX** as classifier **CL**, and also the set of parameters **PAR1**, ..., **PARi** of the feature extractor of the original classifier **CL** may be used. For example, in case classifier **CL** is adapted in-place, no adaptations to this part of the classifier may be needed. Still, this part of the classifier may be independent of the undesired training instances **UTI*.**

As shown in the figure, however, adapting the classifier **CL** may comprise adapting the classification parameters **CPAR**, obtaining adapted parameters **CPAR'**, 366. Shown in the figure are adapted parameters **PDP1'**, 361, up to **PDPk'**, 362, of respective continuous probability distributions of features extracted by the feature extractor **FX**, and adapted prior probabilities **PRP1'**, 363, up to **PRPI'**, 364. Parameters **CPAR'** may be adapted as described for the classifier of Fig. 2. For example, for one, more, or all continuous probability distributions of features, adapted parameters **PDP***' of such a probability distribution may be computed based on current parameters **PDP** of the probability distribution and the one or more undesired training instances **UTI***. The features may however be extracted by feature extractor **FX** in this case. Similarly, adapted prior probabilities **PRP*'** of classes may be computed based on the current prior probabilities **PRP*** of the classes and the one or more undesired training instances **UTI***. The various options discussed for Fig. 2 may be applied here. Interestingly, because of the use of feature extractor, a more expressive classifier **CL** may be obtained, or, looking at it from another way, a smaller classifier **CL** may suffice to reach a certain performance, for example, comprising fewer features. Accordingly, computational or qualitative performance may be improved while still determining a classifier that is independent from the undesired training instances **UTI*.**

Although not shown in the figure, adapted classifier **ACL** may be applied to a query instance by applying the feature extractor **FX** of the adapted classifier **ACL**, for example, the original feature extractor **FX** of the classifier **CL**, to the query instance to obtain a feature representation of the query instance; and applying the adapted classifier **ACL** to the feature representation to obtain a classifier output.

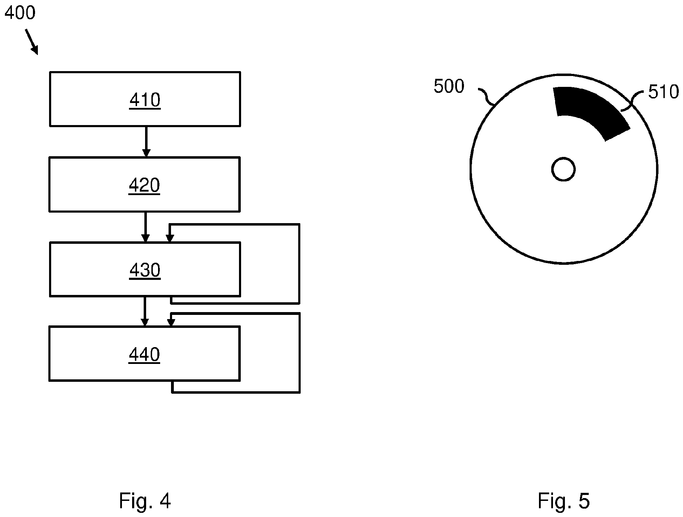

**Fig. 4** shows a block-diagram of computer-implemented method 400 of processing a classifier. The classifier may classify an input instance into multiple classes based on multiple continuous probability distributions of respective features of the input instance and based on prior probabilities of the multiple classes. The method 400 may correspond to an operation of the system 100 of Fig. 1. However, this is not a limitation, in that the method 400 may also be performed using another system, apparatus or device.

The method 400 may comprise, in an operation titled "ACCESSING CLASSIFIER", accessing 410 the classifier.

The method 400 may further comprise, in an operation titled "RECEIVING REMOVAL REQUEST MESSAGE", receiving 420 a removal request message. The removal request message may identify one or more undesired training instances.

The method 400 may further comprise, upon receiving the removal request message, making the classifier independent from the one or more undesired training instances. In order to make the classifier independent from the one or more undesired training instances, the method 400 may comprise, in an operation titled "ADAPTING CONTINUOUS PROBABILITY DISTRIBUTION PARAMETERS", for a continuous probability distribution of a feature, computing 430 adapted parameters of said probability distribution based on current parameters of said probability distribution and the one or more undesired training instances. To make the classifier independent, the method 400 may further comprise, in an operation titled "ADAPTING PRIOR PROBABILITY", computing 440 an adapted prior probability of a class based on a current prior probability of the class and the one or more undesired training instances.

It will be appreciated that, in general, the operations of method 400 of Fig. 4 may be performed in any suitable order, e.g., consecutively, simultaneously, or a combination thereof, subject to, where applicable, a particular order being necessitated, e.g., by input/output relations.

The method(s) may be implemented on a computer as a computer implemented method, as dedicated hardware, or as a combination of both. As also illustrated in **Fig. 5**, instructions for the computer, e.g., executable code, may be stored on a computer readable medium 500, e.g., in the form of a series 510 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 5 shows an optical disc 500.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for processing a classifier, the classifier classifying an input instance into multiple classes based on multiple continuous probability distributions of respective features of the input instance and based on prior probabilities of the multiple classes, the system comprising:
- a data interface (120) configured for accessing the classifier;
- a removal request interface (160) configured for receiving a removal request message, the removal request message identifying one or more undesired training instances;
- a processor subsystem (140) configured to, upon receiving the removal request message, make the classifier independent from one or more undesired training instances by:
- for a continuous probability distribution of a feature, computing adapted parameters of said probability distribution based on current parameters of said probability distribution and the one or more undesired training instances;
- computing an adapted prior probability of a class based on a current prior probability of the class and the one or more undesired training instances.

2. The system (100) of claim 1, wherein the removal request message includes the one or more undesired training instances.

3. The system (100) of claim 2, wherein the processor subsystem (100) is configured to check if an undesired training instance is present in the training dataset by computing a hash of the undesired training instance and checking said presence based on the hash.

4. The system (100) of claim 1, wherein the data interface (120) is further configured for accessing a training dataset on which the classifier is trained, the processor subsystem (140) being configured to retrieving the one or more undesired training instances identified in the removal request message from the training dataset and removing the one or more undesired training instances from the training dataset.

5. The system (100) of claim 4, wherein the processor subsystem (140) is configured to obtain the training dataset by collecting multiple training instances of respective users, the removal request message indicating a user whose training instances are to be removed from the training dataset.

6. The system (100) of any one of the preceding claims, further comprising an anomaly detection system configured to detect that at least one training instance represents an adversarial instance and to send the removal request message for said training instance.

7. The system (100) of any one of the preceding claims, wherein an input instance comprises one or more sensor measurements of a user, for example, an image.

8. The system (100) of claim 7, comprising collecting a training instance of a user by receiving the training instance from a user device, the user device measuring the one or more sensor values as physiological quantities of the user.

9. The system (100) of any one of the preceding claims, wherein the classifier comprises a feature extractor for determining the features of the input instance from the input instance, the feature extractor being trained on a further dataset not comprising the undesired training instance.

10. The system (100) of any one of the preceding claims, wherein a continuous probability distribution is parametrized by one or more moments of the continuous probability distribution.

11. The system (100) of claim 10, wherein the processor subsystem (140) is configured to adapt a mean and variance of the continuous probability distribution, comprising adapting the mean using the current mean and the one or more undesired training instances, and further comprising adapting the variance using the current mean and variance and the one or more undesired training instances.

12. The system (100) of any one of the preceding claims, wherein the processor subsystem (140) is configured to adapt a prior probability of a class based on the current prior probability of the class and the one or more undesired training instances.

13. A computer-implemented method (400) of processing a classifier, the classifier classifying an input instance into multiple classes based on multiple continuous probability distributions of respective features of the input instance and based on prior probabilities of the multiple classes, the computer-implemented method comprising:
- accessing (410) the classifier;
- receiving (420) a removal request message, the removal request message identifying one or more undesired training instances;
- upon receiving the removal request message, making the classifier independent from one or more undesired training instances by:
- for a continuous probability distribution of a feature, computing (430) adapted parameters of said probability distribution based on current parameters of said probability distribution and the one or more undesired training instances;
- computing (440) an adapted prior probability of a class based on a current prior probability of the class and the one or more undesired training instances.

14. The computer-implemented method (400) of claim 13, further comprising obtaining a query instance and applying the adapted classifier to the query instance to obtain a classifier output independent from the one or more undesired training instances.

15. A computer-readable medium (500) comprising transitory or non-transitory data (510) representing instructions which, when executed by a processor system, cause the processor system to perform the computer-implemented method according to claim 13 or 14.
